# EUROPEAN PATENT APPLICATION

(11) **EP 2 107 596 A2**
(43) Date of publication of application: **07.10.2009**
(21) Application number: 09004281.3
(22) Date of filing: 25.03.2009
(51) Int. Cl.: H01K 7/00, H01K 9/00

(54) **Filament lamp**

(30) Priority: 31.03.2008 JP 2008090174
(71) Applicant: Ushiodenki Kabushiki Kaisha, Chiyoda-ku 100 Tokyo (JP)
(72) Inventor: Nakashima, Akinobu, Himeji-shi Hyogo-ken (JP); Tanino, Kenji, Himeji-shi Hyogo-ken (JP)
(74) Representative: Tomerius, Isabel

(57) **Abstract**

To provide a filament lamp having a plurality of filaments disposed together in sequence in the tube axis direction of a light-emitting tube, with miniaturized hermetically sealed parts, by certain sheets of metal foil (26a,26b), of the plurality of metal foils (24a,24b,25a,25b,26a,26b), being disposed out of alignment with respect to the other of the metal foils (24a,24b,25a,25b) in both the tube axis direction as in a direction perpendicular to the tube axis direction.

## Description

### Background of the Invention

### Field of Invention

The present invention relates to a filament lamp, and in particular to a filament lamp used to heat an article to be treated.

### Description of Related Art

Heat treatment devices capable of heating an article to be treated without making contact by optical irradiation from an incandescent lamp or other light source in which filaments are disposed inside a light-emitting tube composed of optically transparent material, for example, are widely used as heat treatment devices in rapid thermal processing (RPT) employed during film formation, oxidation, impurity dispersion, nitriding, film stabilization, silicidation, crystallization, and ion implantation processes that are part of the semiconductor manufacturing process (see, Japanese Unexamined Patent Application JP 7-37833 and Japanese Unexamined Patent Application JP 2002-203804).

In a case in which this kind of optical irradiation type heat treatment device is used to perform RTP on a semiconductor wafer, for example, optical irradiation is performed so that the irradiance onto the entire surface of the semiconductor wafer is uniform. However, at the periphery of the semiconductor wafer, temperature exchange occurs with the peripheral atmosphere, causing a tendency for the temperature to drop as compared with the center region. Since heat is radiated from the lateral surface of the semiconductor wafer, the temperature in the peripheral region of the semiconductor wafer declines, resulting in nonuniformity of temperature distribution on the semiconductor wafer.

In order to solve this problem, the wafer is optically irradiated so as to raise the irradiance on the surface of the periphery of the semiconductor wafer above the irradiance on the surface of the center region of the semiconductor wafer, thereby compensating for the temperature drop caused by thermal radiation from the lateral edge, etc., of the semiconductor wafer, resulting in uniform temperature distribution on the semiconductor wafer.

However, in a conventional heat treatment device, separate control in the lamp tube axis direction has not been possible for tiny specific regions that are extremely small in relation to the light-emitting length of an incandescent lamp. As a result, if optical irradiation is performed at an irradiance attuned to the properties of that specific region, areas other than the specific region are also optically irradiated with the same conditions, making it impossible to perform temperature adjustment for the appropriate temperature conditions in the specific region and in other regions. In other words, it is impossible to control only the irradiance upon a tiny specific region so that temperature conditions are uniform on the article to be treated.

Also, in this specific region, even in a case in which the region is optically irradiated with the same irradiance as other regions, differences occur in the speed of temperature rise. Consequently, the temperature of the specific region is not necessarily the same as the temperature of other regions, leading to an undesired temperature distribution of the treatment temperature on the article to be treated. As a result, it is difficult to impart the desired physical properties to the article to be treated, which is problematic.

Taking note of these circumstances, in a commonly owned application, a filament lamp has been proposed that uses an optical irradiation type heat treatment device having the following configuration as the light source (see, Japanese Unexamined Patent Application JP 2006-279008 and corresponding U.S. Patent Application Publication 2006-279008).

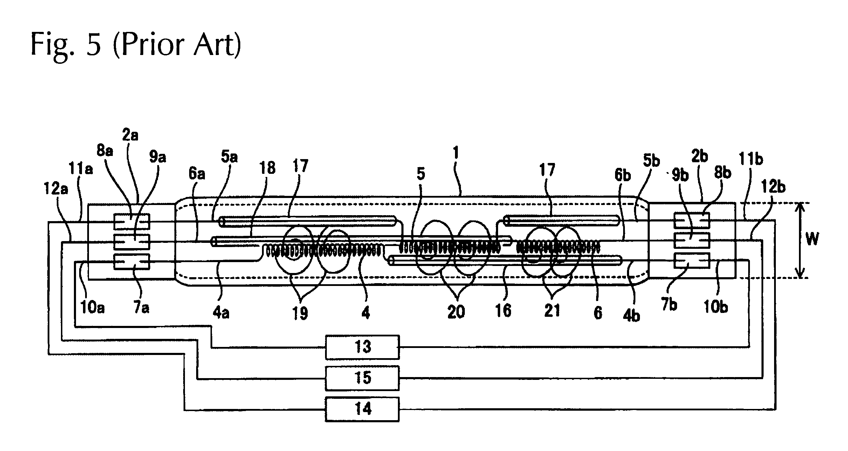

Describing this filament lamp in reference to Fig. 5, coil-shaped filaments 4-6 are disposed together within a straight-tube type light-emitting tube 1 in a sequence extending in the tube axis direction of the light-emitting tube 1, wherein both ends are sealed airtight by hermetically sealed parts 2a. 2b. Internal leads 4a, 4b, 5a, 5b, 6a, 6b for supplying electrical current are connected respectively to both ends of the filaments 4, 5, 6.

The internal leads for the filaments described above extend to the hermetically sealed parts on both ends respectively, and are separately connected electrically to external leads via sheets of metal foil.

In other words, the internal leads 4a, 5a, 6a at one end of the filaments 4, 5, 6, are electrically connected to external leads 10a, 11a, 12a at one end via metal foils 7a, 8a, 9a on the hermetically sealed part 2a. Similarly, the internal leads 4b, 5b, 6b at the other end are electrically connected to external leads 10b, 11b, 12b at the other end via metal foils 7b, 8b, and 9b on the hermetically sealed part 2b.

In addition, each of the filaments 4, 5, and 6 can be supplied with electric current separately by being connected to separate electric current supply devices 13, 14, 15 via the external leads 10a, 10b, 11a, 11b, 12a, 12b.

Insulating tubes 16, 17, 18 are fitted onto the internal leads 4b, 5a, 5b, 6a of the filaments 4, 5, 6, disposed at locations opposite from the filaments 4, 5, 6.

In addition, circular anchors 19, 20, and 21 are disposed at locations between the inner wall of the light-emitting tube 1 and the insulating tubes 16, 17, 18 spaced in the tube axis direction of the light-emitting tube 1. The filaments 4, 5, 6 are each supported by 2 anchors, for example, without making contact with the light-emitting tube 1.

Since electrical current can be supplied separately to a plurality of filaments and control performed separately for illumination, etc., of each filament, using an optical irradiation type heat treatment device using filament lamps of this configuration, optical irradiation is possible with a preferred irradiance according to the properties of the article to be treated, even in a case, for instance, in which the distribution of the degree of localized temperature change on the article to be treated being heat treated is asymmetrical with respect to the shape of the article to be treated. As a result, the article to be treated can be uniformly heated, and as a consequence, uniform temperature distribution can be realized across the entirety of the irradiated surface on the article to be treated, which confers a benefit.

In the conventional technology as described above, the metal foil on the hermetically sealed parts at both ends has a parallel structure, with electric current supplied separately to each of the adjacent sheets of metal foil 7a, 8a, and 9a. Accordingly, a prescribed gap is required between the respective metal foil sheets in order to prevent electric discharge. As a result, the dimension W increases in the perpendicular direction (width direction) relative to the axis direction of the light-emitting tube with the hermetically sealed parts 2a, 2b, and in the case of a heating device in which a plurality of filament lamps is disposed in parallel, the overall size of the device increases, which is problematic. Also, when a plurality of filament lamps is disposed in parallel, the dimension of the space between the lamps is limited by the width dimension of the hermetically sealed parts, making it impossible to dispose lamps in high density, which is problematic.

In addition, in order to obtain high-precision illuminance, the number of filaments within a single filament lamp needs to be increased in order to improve the separate controllability. However, in this case, the number of sheets of metal foil must also be increased in proportion, rendering the problems noted above still more serious.

### Brief Description of the Drawings

Fig. 1 shows a lamp in accordance with a first embodiment of a filament lamp according to the present invention.

Fig. 2 shows a hermetically sealed part arrangement in accordance with a second embodiment of a filament lamp according to the present invention.

Fig. 3 shows a seal arrangement in accordance with a third embodiment of a filament lamp according to the present invention.

Fig. 4 shows a seal arrangement in accordance with a fourth embodiment of a filament lamp according to the present invention.

Fig. 5 shows a conventional lamp.

### Detailed Description of the Invention

Taking note of the problems with the conventional technology described above, the present invention seeks to provide a filament lamp in which the width direction dimension of the hermetically sealed parts does not increase, even if the number of filaments, or in other words, the number of metal foil sheets, increases.

The present invention is characterized by having a plurality of coil-shaped filaments disposed together in sequence in the tube axis direction of a light-emitting tube respectively in the interior of a light-emitting tube having hermetically sealed parts formed at the ends, with a pair of internal leads connected to the ends of each filament and the internal leads being electrically connected to external leads respectively via a plurality of sheets of metal foil; wherein some of the plurality of sheets of metal foil are disposed in staggered relation to the tube axis direction of the light-emitting tube to form a gap at the edges of the metal foil with respect to the other sheets of metal foil, and are disposed staggered in a direction perpendicular to the tube axis.

The filament lamp according to the present invention confers the benefit of reducing the dimension in the direction perpendicular to the axis direction of the light-emitting tube with hermetically sealed parts (the width direction) by disposing the metal foils embedded in the hermetically sealed parts in a staggered manner, with some of the sheets of metal foil disposed in the tube axis direction of the light-emitting tube in relation to other metal foil, and others disposed in a direction perpendicular to the tube axis direction (the width direction). As a result, enlargement of the irradiation device can be avoided, and filament lamps can be disposed in high density.

A first embodiment of the present invention will be described based on drawings with the same reference characters as in Fig. 5 indicating corresponding elements.

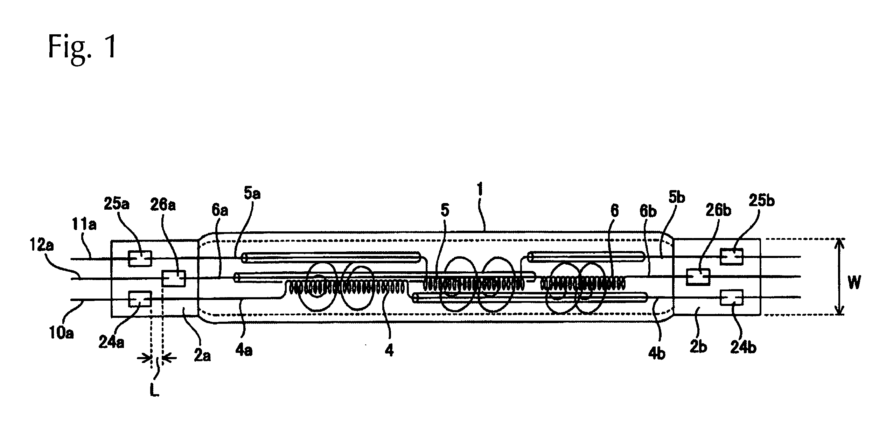

In Fig. 1, some of the sheets of metal foil attached to the internal leads of the filaments are disposed staggered in the tube axis direction of the light-emitting tube in relation to other metal foils, and some are disposed staggered in the direction perpendicular to the tube axis (the width direction).

In other words, at the hermetically sealed part 2a, the metal foil 26a to which the internal lead 6a of filament 6 is attached is disposed staggered in the tube axis direction in relation to the metal foil 24a, to which is attached the internal lead 4a of filament 4, and relative to the metal foil 25a, to which is attached the internal lead 5a of filament 5, forming a gap L between the ends of the metal foils.

Of course, these metal foils 24a, 25a, 26a are also disposed mutually staggered in a direction perpendicular to the tube axis. In other words, the foils are disposed in a zig-zag arrangement. In addition, the metal foils 24b, 25b, 26b in the hermetically sealed part 2b on the opposite end are disposed similarly. By adopting this disposition, the need to create a gap in the width direction between the directly adjacent metal foil 24a and metal foil 26a, and between metal foil 26a and metal foil 25a is eliminated, whereas there was a need to create a prescribed gap in the perpendicular direction to the tube axis for the group of metal foils 7a, 8a, 9a for conventional arrangement shown in Fig. 5. To this extent, the dimension W in the width direction (the direction perpendicular to the tube axis) of the hermetically sealed parts 2a and 2b can be reduced.

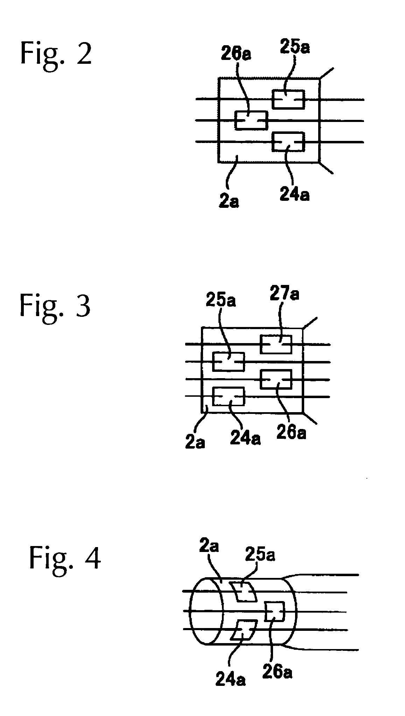

In the embodiment shown in Fig. 1, the metal foils 24a, 25a are disposed at the lamp end of the hermetically sealed part 2a, while the metal foil 26a is disposed in the direction of the light-emitting tube 1. However, as shown in Fig. 2, the metal foil 26a may be disposed at the lamp end, and the metal foils 24a and 25a disposed toward the light-emitting tube 1, i.e., the opposite of the configuration of Fig. 1.

In addition, in the embodiments shown in Figs. 1 & 2, an example was presented of three sheets of metal foil, or in other words, of three filaments. However, the present invention is not limited to this configuration. A case of four or more filaments is acceptable. Fig. 3 shows an example of four filaments, or in other words, having four sheets of metal foil. In this embodiment, the metal foils 24a, 25a are disposed at the lamp ends, while the other two sheets, metal foils 26a, 27a are disposed staggered in the tube axis direction in relation to the others, and staggered in the direction perpendicular to the tube axis, forming a zig-zag configuration.

In addition, the embodiments shown in Figs. 1-3 show an example of employing a pinch seal, but the same configuration can be adopted in a case in which a shrink seal is employed. Fig. 4 shows an example of this case. A detailed configuration of a shrink seal in a multi-filament lamp is described in commonly-owned U.S. Patent Application 2007/120454. The staggering of the disposition of the metal foils 24a, 25a, 26a in the perpendicular direction to the tube axis in this case becomes a staggering in the circular direction of the hermetically sealed part 2a.

The embodiments shown in Figs. 2-4 as described above show only the hermetically sealed part 2a on one end. However, the hermetically sealed part 2b on the other end has the same metal foil disposition structure, of course.

By adopting the metal foil disposition at the hermetically sealed parts as in the present invention, the dimension W in the perpendicular direction to the tube axis for the hermetically sealed part can be reduced, thus avoiding enlargement of the overall device incorporating the lamp and making possible parallel disposition of a plurality of lamps in high density.

## Claims

1. A filament lamp, comprising:
a light-emitting tube having hermetically sealed parts formed at each of opposite ends thereof;
a plurality of coil-shaped filaments disposed within the light-emitting tube in a sequence along a axis of the light emitting tube,
an internal lead connected at each end of each filament and each internal lead being electrically connected to a respective external lead via a sheet of metal foil;
wherein the sheets of metal foil are disposed staggered in relation to the longitudinal axis direction of the light-emitting tube in a manner forming a gap in said longitudinal axis direction between adjacent edges of adjacent metal foils, and wherein the sheets of metal foil are also disposed staggered in a direction perpendicular to the tube axis.
